# EUROPEAN PATENT SPECIFICATION

(11) **EP 4 375 643 B1**
(45) Date of publication and mention of the grant of the patent: **20.08.2025**
(21) Application number: 23209230.4
(22) Date of filing: 10.11.2023
(51) Int. Cl.: G01N 21/39, G01J 3/42, G01J 3/433, G01N 21/03, G01N 21/3504, G01N 33/00

(54) **APPARATUS FOR QUANTITATIVELY DETECTING ISOTOPOLOGUE OF CARBON DIOXIDE USING DUAL-PHOTON ABSORPTION AND SPECTROMETER**
VORRICHTUNG ZUR QUANTITATIVEN DETEKTION VON ISOTOPOLOGEN VON KOHLENDIOXID MITTELS DUAL-PHOTONEN-ABSORPTION UND SPEKTROMETER
APPAREIL DE DÉTECTION QUANTITATIVE D'ISOTOPOLOGUE DE DIOXYDE DE CARBONE UTILISANT UN SPECTROMÈTRE ET UN ABSORPTION À DEUX PHOTONS

(30) Priority: 24.11.2022 CN 202211479911
(43) Date of publication of application: 29.05.2024
(73) Proprietor: Hefei National Laboratory, 230094 Hefei Anhui (CN); University of Science and Technology of China, Anhui 230026 (CN)
(72) Inventor: HU, Shuiming, Hefei, 230026 (CN); CHENG, Cunfeng, Hefei, 230026 (CN)
(74) Representative: Gill Jennings & Every LLP

(56) References cited:
- US-A1- 2018 052 047
- TAN YAN-DONG ET AL: "Detection of radiocarbon dioxide with double-resonance absorption spectroscopy", HUAXUE-WULI-XUEBAO = CHINESE JOURNAL OF CHEMICAL PHYSICS, vol. 34, no. 4, 1 August 2021 (2021-08-01), pages 373 - 380, XP093100435, ISSN: 1674-0068, Retrieved from the Internet <URL:https://pubs.aip.org/cps/cjcp/article-pdf/34/4/373/16745491/373_1_online.pdf> DOI: 10.1063/1674-0068/cjcp2103045
- ZHANG Z.-T. ET AL: "Seeded optical parametric oscillator light source for precision spectroscopy", OPTICS LETTERS, vol. 45, no. 4, 14 February 2020 (2020-02-14), US, pages 1013, XP093147763, ISSN: 0146-9592, Retrieved from the Internet <URL:https://opg.optica.org/view_article.cfm?pdfKey=d308c194-91ad-45d0-9fe4d84c40298d6e_427421> DOI: 10.1364/OL.384582
- FLEISHER ADAM J. ET AL: "Optical Measurement of Radiocarbon below Unity Fraction Modern by Linear Absorption Spectroscopy", JOURNAL OF PHYSICAL CHEMISTRY LETTERS, vol. 8, no. 18, 11 September 2017 (2017-09-11), US, pages 4550 - 4556, XP093100214, ISSN: 1948-7185, DOI: 10.1021/acs.jpclett.7b02105

## Description

The present application claims priority to Chinese Patent Application No. 202211479911.8, titled "APPARATUS FOR QUANTITATIVELY DETECTING ISOTOPOLOGUE OF CARBON DIOXIDE USING DUAL-PHOTON ABSORPTION AND SPECTROMETER ", filed on November 24, 2022 with the China National Intellectual Property Administration.

### FIELD

The present disclosure relates to the technical field of spectral detection, and in particular to a device, which is for quantitatively detecting an isotopologue of carbon dioxide using dual-photon absorption, and a spectrometer.

### BACKGROUND

Carbon dioxide is one of the key greenhouse gases in the earth atmosphere and a key factor in the carbon cycle. Due to the isotope fractionation, content of each isotopologue in a carbon dioxide sample may be quantitatively detected to distinguish a source of the sample, and hence the quantitative detection is widely used in fields such as chronometric dating, tracing, forensic identification, environmental testing, and drug metabolism. Laser spectroscopy is one of important means at present for detecting isotopes in carbon dioxide, and hence has a great potential in the quantitative detection. A main concept of sensitive detection on isotopes in carbon dioxide through the laser spectroscopy is detecting a spectral signal of an isotopologue of carbon dioxide through cavity ring-down spectroscopy.

At present, determination of isotopologues in carbon dioxide through the laser spectroscopy requires higher sensitivity and is subject to apparently insufficient selectivity. Moreover, the laser spectroscopy is limited by the Doppler broadening on spectra linewidth, and hence an isotopologue of carbon dioxide cannot be effectively distinguished from an isotopologue of another carbon compound or another molecule. Hence, it is difficult to achieve accurate quantitative measurement. As an example, "Detection of Radiocarbon Dioxide with Double-Resonance Absorption Spectroscopy" by Yan-dong Tan, et al. (Chinese Journal of Chemical Physics, vol. 34, No. 4, page 373-380) and "Seeded optical parametric oscillator light source for precision spectroscopy" by Zhang Z. -T. et al. (Optics Letters, vol. 45, No. 4, page 1013) discloses a method and a system for detecting radiocarbon dioxide with double-resonance absorption spectroscopy. As another example, "Optical Measurement of Radiocarbon below Unity Fraction Modern by Linear Absorption Spectroscopy" by Fleisher Adam J. et al. discloses a system for measuring radiocarbon by using linear absorption spectroscopy, in which a conventional distributed feedback quantum cascade laser serves as a laser source. As another example, patent document with publication No. US 2018/052047 A1 discloses a carbon isotope analyser, in which a laser beam incident on an optical resonator containing analyte gases yields absorption spectra.

### SUMMARY

The invention is defined in the appended claims.

In view of the above, an apparatus for quantitatively detecting an isotopologue of carbon dioxide using dual-photon absorption and a spectrometer are provided to address at least the above issue.

In a first aspect, an apparatus for quantitatively detecting isotopologue of carbon dioxide using dual-photon absorption are provided according to an embodiment of the present disclosure. The apparatus comprises a laser source, a laser frequency stabilizer, a sample chamber, a signal detector, and a signal analyzer, where: the sample chamber comprises an optical resonator and a piezoelectric ceramic; the laser source is configured to output a laser beam; the laser frequency stabilizer is configured to lock the laser beam to a mode frequency of the optical resonator; the piezoelectric ceramic is configured to adjust a length of the optical resonator to alter the mode frequency of the optical resonator to match energy levels of a target molecular isotopologue; the signal detector is configured to detect a transmission intensity of the light beam passing the optical resonator to obtain a dual-photon absorption signal; and the signal analyzer is configured to analyze and process the dual-photon absorption signal to obtain a concentration of the target molecular isotopologue.

In an embodiment, the laser beam is a continuous infrared laser beam, and a power of the laser beam is greater than 100mW.

In an embodiment, a fineness of the optical resonator is higher than 60000.

The energy levels of the target molecular isotopologue comprise: energy levels of dual-photon absorption, where an energy level representing single-photon absorption is between the energy levels of dual-photon absorption.

Energy of a photon having the mode frequency matching the energy levels of the target molecular isotopologue is equal to a half of an energy difference between the energy levels of the target molecular isotopologue.

In an embodiment, fluctuations of temperature of the optical resonator are less than 10mK.

In an embodiment, the sample chamber further comprises a temperature controller, where the temperature controller is configured to control temperature of the optical resonator.

In an embodiment, the signal detector comprises a detector and a signal amplifier which are integrated, where the detector is configured to detect the transmission intensity of the light beam passing the optical resonator to obtain the dual-photon absorption signal, and the signal amplifier is configured to amplify the obtained dual-photon absorption signal.

In an embodiment, the signal analyzer is configured to: invoke a signal processing program to analyze and process the dual-photon absorption signal to obtain the concentration of the target molecular isotopologue, and display the concentration of the target molecular isotopologue via an interactive interface.

In a second aspect, a spectrometer is further provided according to an embodiment of the present disclosure. The spectrometer comprises the apparatus in any foregoing embodiment.

In comparison with conventional technology, embodiments of the present disclosure achieves at least following beneficial effects.

The apparatus for quantitatively detecting isotopologue of carbon dioxide using dual-photon absorption are provided, comprising the laser source, the laser frequency stabilizer, the sample chamber, the signal detector, and the signal analyzer. The sample chamber comprises the optical resonator and the piezoelectric ceramic. The laser source is configured to output the laser beam. The laser frequency stabilizer is configured to lock the laser beam to the mode frequency of the optical resonator. The piezoelectric ceramic is configured to adjust the length of the optical resonator to alter the mode frequency of the optical resonator to match energy levels of the target molecular isotopologue. The signal detector is configured to detect the transmission intensity of the light beam passing the optical resonator to obtain the dual-photon absorption signal. The signal analyzer is configured to analyze and process the dual-photon absorption signal to obtain the concentration of the target molecular isotopologue. Herein the apparatus operates based on dual-photon absorptiometry. The laser frequency stabilizer controls a frequency of the laser beam to lock the laser beam onto the optical resonator, so that the frequency of the laser beam is consistent with the mode frequency of the optical resonator. The length of the optical resonator is adjusted through the piezoelectric ceramic to tune the mode frequency of the optical resonator, and thereby tune the frequency of the laser beam towards a frequency matching the energy levels of the target molecular isotopologue. The target molecular isotopologue is selectively excited once the frequency of the laser beam matches the energy levels of the target molecular isotopologue. The transmission intensity of the laser beam passing the optical resonator is measured via the signal detector. When an intensity of the laser beam is strong enough, the target molecular isotopologue is capable to absorb two photons, which is reflected in the dual-photon absorption signal. The signal analyzer is configured to analyze and process the dual-photon absorption signal to obtain the concentration of the target molecular isotopologue. A spectral linewidth of the dual-photon absorption is narrow due to its Doppler-free characteristic, which can effectively improve a spectral resolution and selectivity on the isotopologue. Hence, an isotopologue of carbon dioxide can be effectively distinguished in spectra from isotopologues of other carbon compounds and isotopologues of other molecules. Since amplitude of the dual-photon absorption signal is proportional to an intensity of the laser beam outputted from the laser source, the dual-photon absorption signal having sufficient strength can be obtained through increasing the intensity of the transmission light. Accordingly, sensitive quantitative detection of isotopologues of carbon dioxide can be achieved.

### BRIEF DESCRIPTION OF THE DRAWINGS

In order to explain embodiments of the present disclosure or the technical solutions in the conventional technology more clearly, the drawings needed to be used in the description of the embodiments or the conventional technology will be briefly introduced below. Obviously, the drawings in the following description are only embodiments of the present disclosure. For those of ordinary skill in the art, other drawings can be obtained based on the provided drawings without creative efforts.
Figure 1 is a schematic structural diagram of an apparatus for quantitatively detecting isotopologue of carbon dioxide using dual-photon absorption according to an embodiment of the present disclosure.
Figure 2 is a diagram of energy-level matching for an apparatus for quantitatively detecting isotopologue of carbon dioxide using dual-photon absorption according to an embodiment of the present disclosure.
Figure 3 is a schematic structural diagram of an apparatus for quantitatively detecting isotopologue of carbon dioxide using dual-photon absorption according to another embodiment of the present disclosure.
Figure 4 is a schematic structural diagram of an apparatus for quantitatively detecting isotopologue of carbon dioxide using dual-photon absorption according to another embodiment of the present disclosure.
Figure 5 is a schematic structural diagram of an apparatus for quantitatively detecting isotopologue of carbon dioxide using dual-photon absorption according to another embodiment of the present disclosure.
Figure 6 is a schematic diagram of a structure of a spectrometer according to an embodiment of the present disclosure.

### DETAILED DESCRIPTION OF EMBODIMENTS

The technical solutions in the embodiments of the present disclosure will be clearly and completely described below with reference to the accompanying drawings in the embodiments of the present disclosure. Obviously, the described embodiments are only some of the embodiments of the present disclosure, rather than all the embodiments. Based on the embodiments of the present disclosure, all other embodiments obtained by those of ordinary skill in the art without creative efforts fall within the scope of protection of the present disclosure.

In order to clarify and facilitate understanding of the above objects, features and advantages of the present disclosure, hereinafter the present disclosure is further illustrated in detail in conjunction of the drawings and specific embodiments.

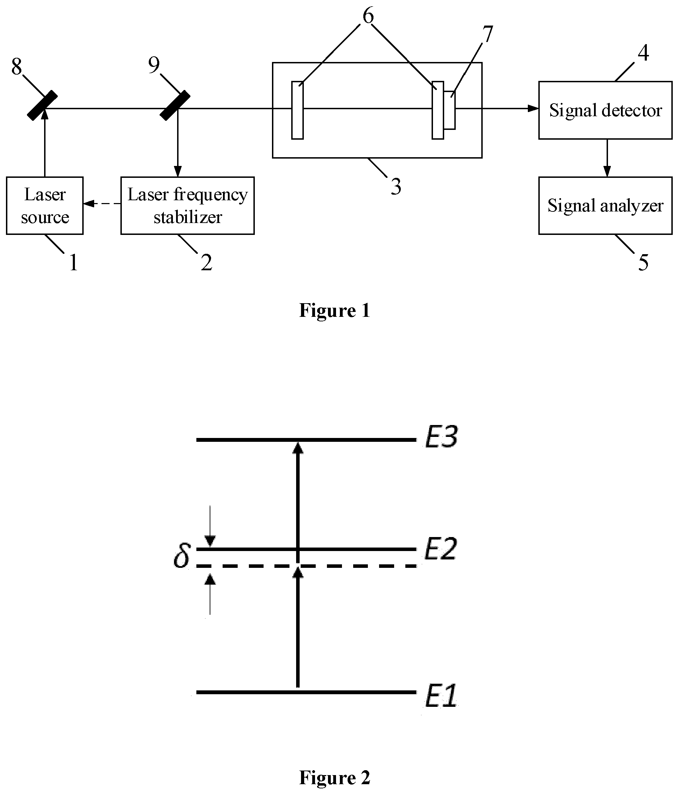

An apparatus for quantitatively detecting isotopologue of carbon dioxide using dual-photon absorption is provided according to an embodiment of the present disclosure. Reference is made to Figure 1, which is a schematic structural diagram of an apparatus for quantitatively detecting an isotopologue of carbon dioxide using dual-photon absorption according to an embodiment of the present disclosure. As shown in Figure 1, the apparatus comprises: a laser source 1, a laser frequency stabilizer 2, a sample chamber 3, a signal detector 4, and a signal analyzer 5. The sample chamber 3 comprises an optical resonator 6 and a piezoelectric ceramic 7.

The laser source 1 is configured to output a laser beam. The laser frequency stabilizer 2 is configured to lock the laser beam to a mode frequency of the optical resonator 6.

In one embodiment, an optical path of the laser beam outputted by the laser source 1 may be deflected via a reflector 8, such that the laser beam is reflected into a beam splitter 9. A part of the laser beam goes into the sample chamber 3 from the beam splitter 9, and another part of the laser beam goes into the laser frequency stabilizer 2. The laser frequency stabilizer 2 may modulate and demodulate a frequency and a phase of the laser beam going into the laser frequency stabilizer 2. The laser frequency stabilizer 2 generates a signal representing an error, generates another signal as a negative feedback of the error, and transmits the negative-feedback signal to the laser source 1, so as to control a frequency of the laser beam emitted by the laser source 1 to be identical to a mode frequency of the optical resonator 6. In addition, the laser frequency stabilizer 2 may further determine automatically whether the laser beam output by the laser source 1 is locked to the mode frequency of the optical resonator 6. In case of negative determination, the laser frequency stabilizer 2 may control the frequency of the laser beam outputted by the laser source 1 automatically, so as to lock the laser beam to the mode frequency of the optical resonator 6.

The piezoelectric ceramic 7 is configured to adjust a length of the optical resonator 6, so as to alter the mode frequency of the optical resonator 6 and thereby match the frequency of the laser beam with energy levels of the target molecular isotopologue.

The signal detector 4 is configured to detect a transmission intensity of the laser beam passing the optical resonator 6, so as to obtain a dual-photon absorption signal.

The signal analyzer 5 is configured to analyze and process the dual-photon absorption signal to obtain a concentration of the target molecular isotopologue.

The apparatus for quantitatively detecting isotopologue of carbon dioxide using dual-photon absorption are provided, comprising the laser source 1, the laser frequency stabilizer 2, the sample chamber 3, the signal detector 4, and the signal analyzer 5. The sample chamber 3 comprises the optical resonator 6 and the piezoelectric ceramic 7. The laser source 1 is configured to output the laser beam. The laser frequency stabilizer 2 is configured to lock the laser beam to the mode frequency of the optical resonator 6. The piezoelectric ceramic 7 is configured to adjust the length of the optical resonator 6 to alter the mode frequency of the optical resonator 6 to match energy levels of the target molecular isotopologue. The signal detector 4 is configured to detect the transmission intensity of the light beam passing the optical resonator 6 to obtain the dual-photon absorption signal. The signal analyzer 5 is configured to analyze and process the dual-photon absorption signal to obtain the concentration of the target molecular isotopologue. Herein the apparatus operates based on dual-photon absorptiometry. The laser frequency stabilizer 2 controls a frequency of the laser beam to lock the laser beam onto the optical resonator 6, so that the frequency of the laser beam is consistent with the mode frequency of the optical resonator 6. The length of the optical resonator 6 is adjusted through the piezoelectric ceramic 7 to tune the mode frequency of the optical resonator 6, and thereby tune the frequency of the laser beam towards a frequency matching the energy levels of the target molecular isotopologue. The target molecular isotopologue is selectively excited once the frequency of the laser beam matches the energy levels of the target molecular isotopologue. The transmission intensity of the laser beam passing the optical resonator 6 is measured via the signal detector 4. When an intensity of the laser beam is strong enough, the target molecular isotopologue is capable to absorb two photons, which is reflected in the dual-photon absorption signal. The signal analyzer 5 is configured to analyze and process the dual-photon absorption signal to obtain the concentration of the target molecular isotopologue. A spectral linewidth of the dual-photon absorption is narrow due to its Doppler-free characteristic, which can effectively improve a spectral resolution and selectivity on the isotopologue. Hence, an isotopologue of carbon dioxide can be effectively distinguished in spectra from isotopologues of other carbon compounds and isotopologues of other molecules. Since amplitude of the dual-photon absorption signal is proportional to a square of an intensity of transmission light after the excitation, the dual-photon absorption signal having sufficient strength can be obtained through increasing the intensity of the transmission light. Accordingly, sensitive quantitative detection of isotopologues of carbon dioxide can be achieved.

Hereinafter a structure of the foregoing apparatus for quantitatively detecting isotopologue of carbon dioxide using dual-photon absorption is further illustrated in details in conjunction with optional embodiments.

In an embodiment, the laser source 1 is an infrared laser source. The laser beam is a continuous infrared laser beam, and a power of the laser beam may be greater than 100mW.

In an embodiment, the optical resonator 6 is a high-fineness optical resonator. For example, fineness of the optical resonator 6 is higher than 60,000.

In an embodiment, a wavelength of the laser beam outputted by the laser source 1 can be rapidly tuned. A tunable bandwidth of the wavelength of the laser beam may be 1MHz preferably, and the fineness of the optical resonator 6 for a single laser frequency is higher than 60,000.

In an embodiment, the energy levels of the target molecular isotopologue comprise energy levels of dual-photon absorption. An energy level representing single-photon absorption is between the energy levels of dual-photon absorption.

In an embodiment, the mode frequency matching the energy levels of the target molecular isotopologue refers to that energy of a photon having the mode frequency is equal to, or substantially equal to, a half of an energy difference between the energy levels of the target molecular isotopologue.

In an embodiment, the energy levels of the target molecular isotopologue are energy levels which are selected. Reference is made to Figure 2, which is a diagram of energy-level matching for an apparatus for quantitatively detecting an isotopologue of carbon dioxide using dual-photon absorption according to an embodiment of the present disclosure. In Figure 2, E1 and E3 denote the energy levels of dual-photon absorption, and E2 denotes an energy level representing (i.e., relating to) single-photon absorption. E2 is located approximately at the middle between E1 and E3, that is, the single-photon absorption takes places under energy approximately half of energy required for the dual-photon absorption. A difference between the energy level representing single-photon absorption and a right middle position between the energy levels of dual-photon absorption does not exceed δ. Moreover, when the frequency of the laser beam outputted by the laser source 1 is controlled to be a frequency corresponding a half of the energy difference between the energy levels of the target molecular isotopologue, the frequency of the laser beam is close to the energy level representing single-photon absorption. In such case, strength of the dual-photon absorption signal can be greatly improved.

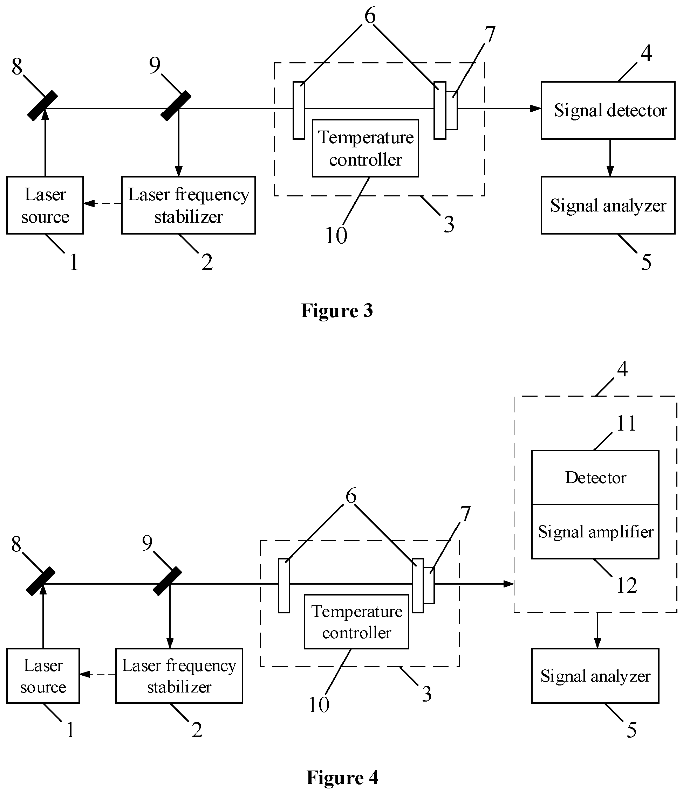

Hereinafter a structure of the sample chamber 3 in the foregoing apparatus for quantitatively detecting the isotopologue of carbon dioxide using dual-photon absorption is further illustrated in conjunction with optional embodiments. Reference is made to Figure 3, which illustrates a schematic structural diagram of an apparatus for quantitatively detecting an isotopologue of carbon dioxide using dual-photon absorption according to another embodiment. As shown in Figure 3, the sample chamber 3 further comprises a temperature controller 10, which is configured to control temperature of the optical resonator 6.

In an embodiment, fluctuations of the temperature of the optical resonator 6 are within 10mK.

In an embodiment, a means of keeping the fluctuations of the temperature of the optical resonator 6 within 10 mK includes but is not limited to controlling the optical resonator 6 through the temperature controller 10 K. Other means of temperature adjustment may also feasible for controlling the temperature of the optical resonator 6 to fluctuate within 10mK.

Hereinafter a structure of the signal detector 4 in the foregoing apparatus for quantitatively detecting the isotopologue of carbon dioxide using dual-photon absorption is further illustrated in conjunction with optional embodiments. Reference is made to Figure 4, which illustrates a schematic structural diagram of an apparatus for quantitatively detecting an isotopologue of carbon dioxide using dual-photon absorption according to another embodiment. As shown in Figure 4, the signal detector 4 comprises a detector 11 and a signal amplifier 12 which are integrated.

The detector 11 may be a sensitive detector. In an embodiment, the detector is configured to detect a transmission intensity of the laser beam passing the optical resonator 6, so as to obtain the dual-photon absorption signal.

The signal amplifier 12 may be a low-noise signal amplifier. In an embodiment, the signal amplifier 12 is configured to amplify the obtained dual-photon absorption signal.

In an embodiment, the target molecular isotopologue can be selectively excited when the frequency of the laser beam outputted by the laser source 1 matches the energy levels of the target molecular isotopologue, and the light beam passes the optical resonator 6 as a transmitting beam after the target molecular isotopologue is excited and then is detected by the detector 11. Thereby, the dual-photon absorption signal is obtained. Since the dual-photon absorption signal detected by the signal detector 4 is weak, the obtained dual-photon absorption signal is first amplified by the low-noise signal amplifier 12, which facilitates subsequent processing of the dual-photon absorption signal.

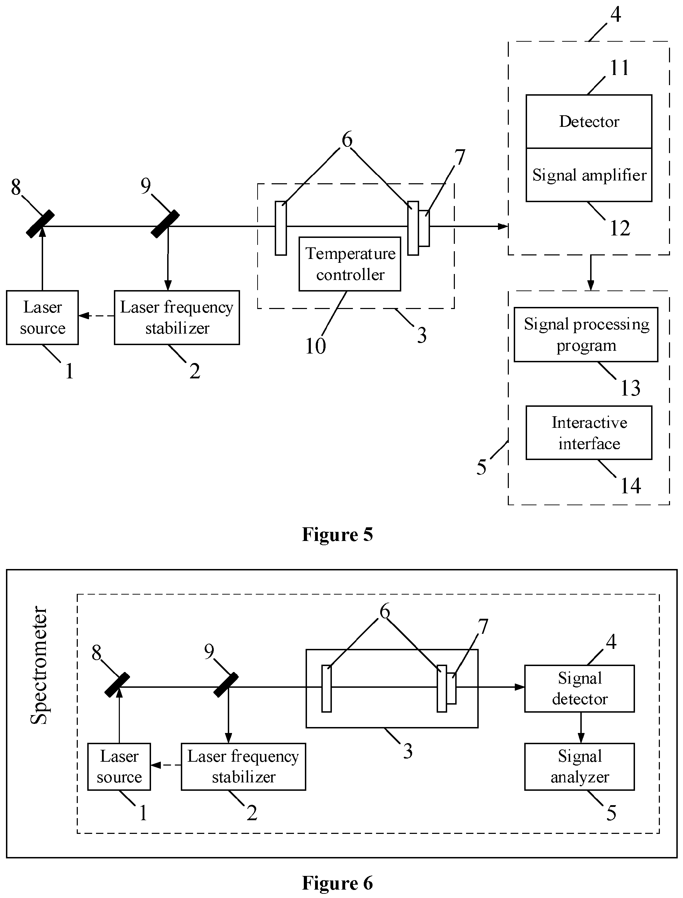

Hereinafter a structure of the signal analyzer 5 in the foregoing apparatus for quantitatively detecting the isotopologue of carbon dioxide using dual-photon absorption is further illustrated in conjunction with optional embodiments. Reference is made to Figure 5, which illustrates a schematic structural diagram of an apparatus for quantitatively detecting an isotopologue of carbon dioxide using dual-photon absorption according to another embodiment. As shown in Figure 5, the signal analyzer 5 may comprise a storage medium storing a signal processing program 13, and provide an interactive interface 14. The signal analyzer 5 is configured to invoke the signal processing program 13 to analyze and process the dual-photon absorption signal to obtain the concentration of the target molecular isotopologue, and display the concentration of the target molecular isotopologue via an interactive interface 14.

In an embodiment, the interactive interface 14 may update the displayed concentration of the target molecular isotopologue in real time, and operations may be performed on the displayed concentration of the target molecular isotopologue via the interactive interface 14. The interactive interface 14 comprises but is not limited to an electronic device such as a computer and a mobile phone.

On a basis of the foregoing embodiments, a spectrometer is further provided according to an embodiment of the present disclosure. Reference is made to Figure 6, which is a schematic diagram of a spectrometer according to an embodiment of the present disclosure. The spectrometer comprises the apparatus for quantitatively detecting the isotopologue of carbon dioxide by the dual-photon absorption as described in any foregoing embodiment.

In some embodiments, the spectrometer has the same features as the apparatuses in the foregoing embodiments.

In addition, it is noted that the apparatus and the spectrometer as described in the foregoing embodiments of the present disclosure is not limited to quantitative detection on isotopologue(s) of carbon dioxide, and may be utilized to detect isotopologue(s) of another molecule.

Hereinabove the apparatus for quantitatively detecting the isotopologue of carbon dioxide by dual-photon absorption and the spectrometer are illustrated in detail according to embodiments of the present disclosure. Specific examples are applied herein to illustrate the principles and embodiments of the present disclosure. Description of the above embodiments is only intended for helping understand technical solutions and a core concept of the present disclosure. Those of ordinary skill in the art can make changes in specific implementations and application scopes according to the concept of the present disclosure. In summary, content of this specification should not be construed as a limitation of the present disclosure.

The embodiments of the present disclosure are described in a progressive manner, and each embodiment places emphasis on the difference from other embodiments. Therefore, one embodiment can refer to other embodiments for the same or similar parts. Since the spectrometer disclosed in the embodiments corresponds to the apparatus disclosed in the embodiments, the description of the spectrometer is simple, and reference may be made to the relevant part of the apparatus.

It should be noted that, the relationship terms such as "first", "second" and the like are only used herein to distinguish one entity or operation from another, rather than to necessitate or imply that an actual relationship or order exists between the entities or operations. Furthermore, the terms such as "include", "comprise" or any other variants thereof means to be non-exclusive. Therefore, a process, a method, an article or a device including a series of elements include not only the disclosed elements but also other elements that are not clearly enumerated, or further include inherent elements of the process, the method, the article or the device. Unless expressively limited, the statement "including a..." does not exclude the case that other similar elements may exist in the process, the method, the article or the device other than enumerated elements.

According to the description of the disclosed embodiments, those skilled in the art can implement or use the present disclosure. Various modifications made to these embodiments may be obvious to those skilled in the art, and the general principle defined herein may be implemented in other embodiments without departing from the scope of the present disclosure. Therefore, the present disclosure is not limited to the embodiments described herein but is only limited by the appended claims.

## Claims

1. An apparatus for quantitatively detecting isotopologue of carbon dioxide using dual-photon absorption, comprising:
a laser source (1), a laser frequency stabilizer (2), a sample chamber (3), a signal detector (4), and a signal analyzer (5), wherein:
the sample chamber (3) comprises an optical resonator (6) and a piezoelectric ceramic (7);
the laser source (1) is configured to output a laser beam;
the laser frequency stabilizer (2) is configured to lock the laser beam to a mode frequency of the optical resonator (6);
the piezoelectric ceramic (7) is configured to adjust a length of the optical resonator (6) to alter the mode frequency of the optical resonator (6) to match energy levels of a target molecular isotopologue;
the signal detector (4) is configured to detect a transmission intensity of the light beam passing the optical resonator (6) to obtain a dual-photon absorption signal; and
the signal analyzer (5) is configured to analyze and process the dual-photon absorption signal to obtain a concentration of the target molecular isotopologue;
wherein the energy levels of the target molecular isotopologue comprise energy levels of dual-photon absorption, and an energy level representing single-photon absorption is between the energy levels of dual-photon absorption;
**characterized in that**
energy of a photon having the mode frequency matching the energy levels of the target molecular isotopologue is equal to a half of an energy difference between the energy levels of the target molecular isotopologue.

2. The apparatus according to claim 1, wherein the laser beam is a continuous infrared laser beam, and a power of the laser beam is greater than 100mW.

3. The apparatus according to claim 1 or claim 2, wherein a fineness of the optical resonator (6) is higher than 60000.

4. The apparatus according to any of the preceding claims, wherein fluctuations of temperature of the optical resonator (6) are less than 10mK.

5. The apparatus according to any of the preceding claims, wherein the sample chamber (3) further comprises:
a temperature controller (10), configured to control temperature of the optical resonator (6).

6. The apparatus according to any of the preceding claims, wherein:
the signal detector (4) comprises a detector (11) and a signal amplifier (12) which are integrated;
the detector (11) is configured to detect the transmission intensity of the light beam passing the optical resonator (6) to obtain the dual-photon absorption signal;
the signal amplifier (12) is configured to amplify the obtained dual-photon absorption signal.

7. The apparatus according to any of the preceding claims, wherein the signal analyzer (5) is configured to:
invoke a signal processing program to analyze and process the dual-photon absorption signal to obtain the concentration of the target molecular isotopologue, and
display the concentration of the target molecular isotopologue via an interactive interface.

8. A spectrometer, comprising the apparatus according to any one of claims 1 to 7.

## Patentansprüche

1. Einrichtung zum quantitativen Erfassen des Isotopologs von Kohlendioxid unter Verwendung von Dual-Photonen-Absorption, umfassend:
eine Laserquelle (1), einen Laserfrequenzstabilisator (2), eine Probenkammer (3), einen Signaldetektor (4) und einen Signalanalysator (5), wobei:
die Probenkammer (3) einen optischen Resonator (6) und eine piezoelektrische Keramik (7) umfasst;
die Laserquelle (1) dazu konfiguriert ist, einen Laserstrahl auszugeben;
der Laserfrequenzstabilisator (2) dazu konfiguriert ist, den Laserstrahl auf eine Modenfrequenz des optischen Resonators (6) zu verriegeln;
die piezoelektrische Keramik (7) dazu konfiguriert ist, eine Länge des optischen Resonators (6) anzupassen, um die Modenfrequenz des optischen Resonators (6) zu ändern, um mit den Energieniveaus eines molekularen Zielisotopologs übereinzustimmen;
der Signaldetektor (4) dazu konfiguriert ist, eine Übertragungsintensität des Lichtstrahls zu erfassen, der den optischen Resonator (6) passiert, um ein Dual-Photonen-Absorptionssignal zu erhalten; und
der Signalanalysator (5) dazu konfiguriert ist, das Dual-Photonen-Absorptionssignal zu analysieren und zu verarbeiten, um eine Konzentration des molekularen Zielisotopologs zu erhalten;
wobei die Energieniveaus des molekularen Zielisotopologs Energieniveaus der Dual-Photonen-Absorption umfassen, und ein Energieniveau, das die Einzel-Photonen-Absorption darstellt, zwischen den Energieniveaus der Dual-Photonen-Absorption liegt;
**dadurch gekennzeichnet, dass** Energie eines Photons mit der Modenfrequenz, die mit den Energieniveaus des molekularen Zielisotopologs übereinstimmt, gleich einer Hälfte einer Energiedifferenz zwischen den Energieniveaus des molekularen Zielisotopologs ist.

2. Einrichtung nach Anspruch 1, wobei der Laserstrahl ein kontinuierlicher Infrarot-Laserstrahl ist und eine Leistung des Laserstrahls größer als 100 mW ist.

3. Einrichtung nach Anspruch 1 oder 2, wobei eine Feinheit des optischen Resonators (6) höher als 60000 ist.

4. Einrichtung nach einem der vorhergehenden Ansprüche, wobei die Temperaturschwankungen des optischen Resonators (6) weniger als 10 mK sind.

5. Einrichtung nach einem der vorhergehenden Ansprüche, wobei die Probenkammer (3) ferner umfasst:
eine Temperatursteuervorrichtung (10), die dazu konfiguriert ist, die Temperatur des optischen Resonators (6) zu steuern.

6. Einrichtung nach einem der vorhergehenden Ansprüche, wobei:
der Signaldetektor (4) einen Detektor (11) und einen Signalverstärker (12) umfasst, die integriert sind;
der Detektor (11) dazu konfiguriert ist, die Übertragungsintensität des Lichtstrahls zu erfassen, der den optischen Resonator (6) passiert, um das Dual-Photonen-Absorptionssignal zu erhalten;
der Signalverstärker (12) dazu konfiguriert ist, das erhaltene Dual-Photonen-Absorptionssignal zu verstärken.

7. Einrichtung nach einem der vorhergehenden Ansprüche, wobei der Signalanalysator (5) konfiguriert ist, zum:
Aufrufen eines Signalverarbeitungsprogramms zum Analysieren und Verarbeiten des Dual-Photonen-Absorptionssignals, um die Konzentration des molekularen Zielisotopologs zu erhalten, und
Anzeigen der Konzentration des molekularen Zielisotopologs über eine interaktive Schnittstelle.

8. Spektrometer, umfassend die Einrichtung nach einem der Ansprüche 1 bis 7.

## Revendications

1. Appareil de détection quantitative d'isotopologue de dioxyde de carbone utilisant l'absorption à deux photons, comprenant :
une source laser (1), un stabilisateur de fréquence laser (2), une chambre d'échantillon (3), un détecteur de signal (4) et un analyseur de signal (5), dans lequel :
la chambre d'échantillon (3) comprend un résonateur optique (6) et une céramique piézoélectrique (7) ;
la source laser (1) est configurée pour sortir un faisceau laser ;
le stabilisateur de fréquence laser (2) est configuré pour verrouiller le faisceau laser à une fréquence de mode du résonateur optique (6) ;
la céramique piézoélectrique (7) est configurée pour ajuster une longueur du résonateur optique (6) afin de modifier la fréquence de mode du résonateur optique (6) pour correspondre à des niveaux d'énergie d'un isotopologue moléculaire cible ;
le détecteur de signal (4) est configuré pour détecter une intensité de transmission du faisceau lumineux passant le résonateur optique (6) afin d'obtenir un signal d'absorption à deux photons ; et
l'analyseur de signal (5) est configuré pour analyser et traiter le signal d'absorption à deux photons afin d'obtenir une concentration de l'isotopologue moléculaire cible ;
dans lequel les niveaux d'énergie de l'isotopologue moléculaire cible comprennent des niveaux d'énergie de l'absorption à deux photons, et un niveau d'énergie représentant l'absorption à un seul photon est entre les niveaux d'énergie de l'absorption à deux photons ;
**caractérisé en ce que**
l'énergie d'un photon ayant la fréquence de mode correspondant aux niveaux d'énergie de l'isotopologue moléculaire cible est égale à une moitié d'une différence d'énergie entre les niveaux d'énergie de l'isotopologue moléculaire cible.

2. Appareil selon la revendication 1, dans lequel le faisceau laser est un faisceau laser infrarouge continu, et une puissance du faisceau laser est supérieure à 100 mW.

3. Appareil selon la revendication 1 ou la revendication 2, dans lequel une finesse du résonateur optique (6) est supérieure à 60000.

4. Appareil selon l'une quelconque des revendications précédentes, dans lequel des fluctuations de température du résonateur optique (6) sont inférieures à 10 mK.

5. Appareil selon l'une quelconque des revendications précédentes, dans lequel la chambre d'échantillon (3) comprend en outre :
un contrôleur de température (10), configuré pour contrôler la température du résonateur optique (6).

6. Appareil selon l'une quelconque des revendications précédentes, dans lequel :
le détecteur de signal (4) comprend un détecteur (11) et un amplificateur de signal (12) qui sont intégrés ;
le détecteur (11) est configuré pour détecter l'intensité de transmission du faisceau lumineux passant le résonateur optique (6) afin d'obtenir le signal d'absorption à deux photons ;
l'amplificateur de signal (12) est configuré pour amplifier le signal d'absorption à deux photons obtenu.

7. Appareil selon l'une quelconque des revendications précédentes, dans lequel l'analyseur de signal (5) est configuré pour :
appeler un programme de traitement de signal pour analyser et traiter le signal d'absorption à deux photons afin d'obtenir la concentration de l'isotopologue moléculaire cible, et
afficher la concentration de l'isotopologue moléculaire cible via une interface interactive.

8. Spectromètre, comprenant l'appareil selon l'une quelconque des revendications 1 à 7.
